**Europäisches Patentamt**

**European Patent Office**

**Office européen des brevets**

(19)

(11) Veröffentlichungsnummer: **0 105 089**
**B1**

(12) # EUROPÄISCHE PATENTSCHRIFT

(45) Veröffentlichungstag der Patentschrift:
21.08.85

(51) Int. Cl.⁴: **B 01 F 3/04**, B 01 F 3/02,
C 01 C 1/02, C 07 C 31/04,
B 01 J 10/00, B 01 J 19/00

(21) Anmeldenummer: 83106036.3

(22) Anmeldetag: 21.06.83

(54) Verfahren zum Befeuchten eines Gasstromes, insbesondere für Methanol- und/oder Ammoniakanlagen.

(30) Priorität: 01.10.82 DE 3236441

(43) Veröffentlichungstag der Anmeldung:
11.04.84 Patentblatt 84/15

(45) Bekanntmachung des Hinweises auf die Patenterteilung:
21.08.85 Patentblatt 85/34

(84) Benannte Vertragsstaaten:
AT DE FR GB IT NL SE

(56) Entgegenhaltungen:
DE - B - 1 253 679
US - A - 3 958 956

(73) Patentinhaber: Uhde GmbH, Friedrich-Uhde-Strasse 15,
D-4600 Dortmund 1 (DE)

(72) Erfinder: Ilgner, Hartmut, Dipl.-Ing., Am Siepenhohl 14,
D-4600 Dortmund 30 (DE)
Erfinder: Kledewski, Peter, Dipl.-Ing., Auf der Kluse 16,
D-4600 Dortmund 30 (DE)
Erfinder: Heun, Reinhard, Dipl.-Ing., Fliederweg 11,
D-5804 Herdecke (DE)

(74) Vertreter: Patentanwälte Meinke und Dabringhaus
Dipl.-Ing. J. Meinke Dipl.-Ing. W. Dabringhaus,
Westenhellweg 67, D-4600 Dortmund 1 (DE)

## Beschreibung

Die Erfindung richtet sich auf ein Verfahren zum Befeuchten eines Gasstromes, bei dem aufgeheiztes Wasser zur Abgabe von Wasserdampf im Gegenstrom zum Gasstrom durch einen Gasbefeuchter und anschließend nach Einspeisung von der Menge des abgegebenen Wasserdampfes entsprechendem Zusatzwasser einer Aufheizstufe und zurück zum Gasbefeuchter im Kreislauf geführt wird.

Ein derartiges Verfahren ist bekannt. Dieses Verfahren wird z. B. bei der Erdgasaufbereitung eingesetzt. Bei derartigen katalytischen Prozessen ist es manchmal nötig, ein bestimmtes Wasserdampf-/Gasverhältnis zu erreichen, was beim Stand der Technik durch ein Verfahren der eingangs bezeichneten Art erreicht wird, wobei die Aufnahme von Wasserdampf dort nicht ausreichend genug ist, so daß energetisch hochwertiger Dampf in den Gasstrom nach Verlassen des Gasbefeuchters noch zusätzlich eingespeist werden muß.

Aufgabe der Erfindung ist die Schaffung einer Lösung, mit der eine Gasbefeuchtung vorgenommen werden kann, deren Wasseraufnahme besonders hoch ist und den späteren Einsatzbedingungen möglichst nahekommt, gleichzeitig aber qualitativ mindere Energie, insbesondere Energie auf niedrigem Temperaturniveau, ausgenutzt werden kann.

Mit einem Verfahren der eingangs bezeichneten Art wird diese Aufgabe gemäß der Erfindung dadurch gelöst, daß Teilmengen des Kreislaufwassers in mehreren, hintereinanderliegenden Stufen dem Gasbefeuchter entnommen und die einzelnen Teilmengen einzeln und/oder gemeinsam erhitzt bzw. erwärmt werden.

Durch die Erfindung wird erreicht, daß nicht mehr der Gesamtwasserstrom von der den Gasbefeuchter verlassenden niedrigen Temperatur auf die höhere Eintrittstemperatur erhitzt zu werden braucht, sondern hierfür eine Stufenerhitzung einzelner Teilströme nutzbar gemacht werden kann.

In Ausgestaltung sieht die Erfindung vor, daß das Kreislaufwasser in zwei Stufen dem Gasbefeuchter entnommen wird, wobei die erste entnommene Teilmenge größer ist als die zweite Teilmenge. Grundsätzlich könnten auch andere Mengenverhältnisse, etwa gleich große Teilströme, vorgesehen sein, allerdings ist die erfindungsgemäße Aufteilung der Teilmengen besonders zweckmäßig.

Vorteilhaft ist es, wenn, wie dies die Erfindung ebenfalls vorsieht, die zweite Teilmenge einer Vorwärmstufe und anschließend zusammen mit der ersten Teilmenge einer Endheizstufe zugeführt wird. Diese Verfahrensweise ermöglicht es, die entsprechenden Vorwärm- bzw. Endheizstufen in günstigen Temperatur-Bereichen auslegen zu können, so daß bereits geringere Temperaturdifferenzen ausreichen, die Durchsatzmengen zu erwärmen.

In weiterer Ausgestaltung ist nach der Erfindung vorgesehen, daß der letzten aus dem Gasbefeuchter entnommenen Teilmenge die Zusatzwassermenge vor der ersten Vorwärmstufe zugeführt wird.

Um besonders Abfallwärme, z. B. die Synthesegaswärme, aus einem Methanolreaktor zum Erwärmen des Kreislaufwassers optimal ausnützen zu können, ist nach der Erfindung vorgesehen, daß die Vorwärmstufe oder -stufen und die Endheizstufe als Wärmetauscher für heißes Synthesegas hintereinander in einen gemeinsamen Wärmestrom geschaltet sind, wobei in weiterer Ausgestaltung vorgesehen sein kann, daß die Teilmenge aus den vorangehenden, stromabwärts bezogen auf den Wärmestrom liegenden Wärmetauscher zusammen mit einer Teilmenge aus dem Gasbefeuchter den nächsten stromaufwärts liegenden Wärmetauscher zugeführt wird.

Diese Ausgestaltungen der erfindungsgemäßen Verfahrensweise sind besonders zweckmäßig, da die entsprechend eingesetzten, dem Wärmestrom ausgesetzten Wärmetauscher sehr günstig ausgelegt werden können, so daß z. B. die Restwassermenge aus dem Sumpf des Gasbefeuchters dem am weitesten stromabwärts liegenden Wärmetauscher, d. h. dem kältesten Wärmetauscher, zur ersten Vorheizung zugeführt wird, sodann wird die an vorletzter Stelle entnommene Teilmenge von aufzuwärmendem Wasser aus dem Gasbefeuchter zusammen mit der der ersten Vorwärmung unterzogenen Restmenge dem nächsten Wärmetauscher zugeführt. Die Einrichtung wird dann zweckmäßig so getroffen, daß jeweils die aus einer Stufe des Gasbefeuchters entnommene Teilmenge im gleichen Temperaturbereich liegt, wie die dem vorangegangenen Vorwärmer verlassende und dieser Teilmenge zugemischte andere Teilmenge.

Die Erfindung ist nachstehend anhand der Zeichnung näher beschrieben. Dabei zeigt

Fig. 1 ein Prinzipschaltbild nach dem Stand der Technik,

Fig. 2 ein Prinzipschaltbild nach einem Ausführungsbeispiel der Erfindung und

Fig. 3 ein weiteres Ausführungsbeispiel der Erfindung.

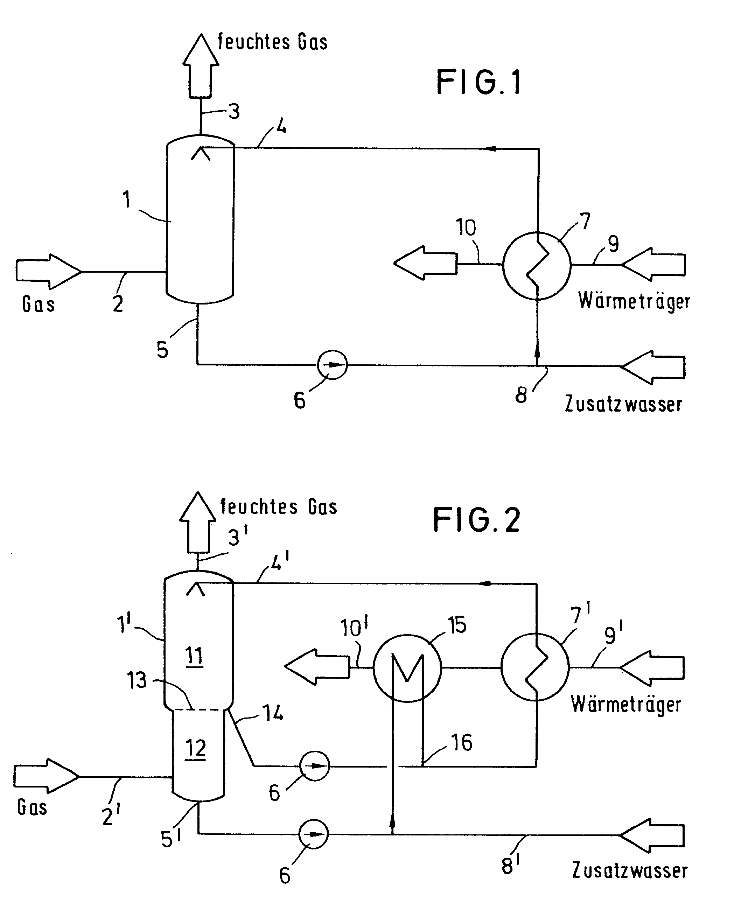

In Fig. 1, die den Stand der Technik, der Ausgangspunkt der vorliegenden Erfindung bildet, darstellt, wird einem Gasbefeuchter 1 im unteren Bereich das mit Wasserdampf zu versehene Gas bei 2 zugeführt. Das mit Wasserdampf angereicherte Gas verläßt oben den Gasbefeuchter bei 3. Aufgeheiztes Wasser wird oben in dem Gasbefeuchter 1 bei 4 eingegeben und unten im Sumpf bei 5 abgezogen, wobei die eingesprühte Heißwassermenge um den Betrag größer ist, als die aus dem Sumpf abgezogene, um den das Gas mit Wasserdampf angereichert worden ist. Das durch den Gasgegenstrom abgekühlte im Sumpf gesammelte Wasser 5 wird über eine Pumpe 6 einem Wärmetauscher 7 zugeführt, wobei vor dem Wärmetauscher 7 bei 8 eine Menge an Zusatzwasser in den Kreislauf eingespeist wird, die der Menge des vom Gas aufgenommenen Wasserdampfes entspricht. Vom Wärmetauscher 7 wird dann das erhitzte Heißwasser wieder bei 4 in den Gasbefeuchter 1 eingesprüht. Der Wärmetauscher 7 wird z. B. von einem Wärmestrom, etwa einem heißen Synthesegasstrom aus einem Methanolreaktor beheizt, was durch die Zuleitung 9 zum Wärmetauscher und die Ableitung 10 mit den entsprechenden Pfeilen angedeutet ist.

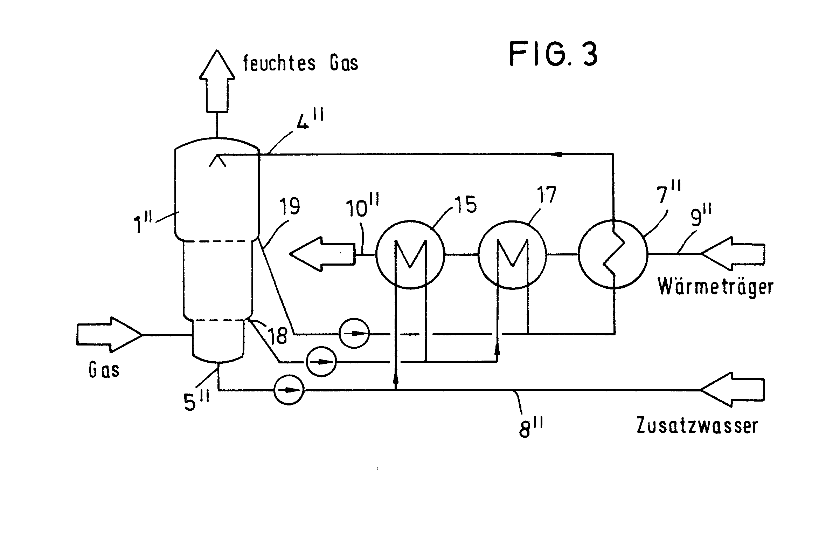

Bei der Beschreibung der Erfindung nach den Fig. 2 und 3 werden die dem Prinzip nach gleichen Anlageelemente mit gleichen Bezugsziffern bezeichnet. Zur besseren Identifizierung allerdings einfach bzw. zweifach gestrichen. Dies bedeutet z. B., daß der Gasbefeuchter in Fig. 2 mit dem Bezugszeichen 1' bezeichnet ist, der Gasbefeuchter in Fig. 3 mit dem Bezugszeichen 1''.

Wie sich bei dem Ausführungsbeispiel aus Fig. 2 ergibt, ist dort der Gasbefeuchter prinzipiell in zwei Bereiche eingeteilt; in einen oberen Bereich 11 und einen unteren Bereich 12. In dem oberen Bereich 11 wird die Gesamtmenge des heißen eingesetzten Prozeßwassers bei 4' eingesprüht und beaufschlagt die Gesamtmenge des austretenden Gasstromes, der unten bei 2' den Gasbefeuchter beaufschlagt und oben bei 3' mit Wasserdampf befrachtet verläßt.

Durch eine gestrichelt wiedergegebene Trennlinie 13 im Gasbefeuchter 1' ist die Bereichstrennung zwischen 11 und 12 angedeutet. An dieser Stelle wird eine Teilmenge des Prozeßwassers bei 14 abgezogen. Die Restmenge beaufschlagt noch den unteren Bereich 12 des Gasbefeuchters 1' und tritt im Sumpf bei 5' aus. Die bei 14 ausgetretene, erste Teilmenge weist eine höhere Temperatur auf als die aus dem Sumpf austretende zweite Teilmenge bzw. Restmenge, wobei die erste Teilmenge beim Ausführungsbeispiel größer sein soll als die Restmenge, was durch die unterschiedlichen Proportionen der Bereiche 11 und 12 des Gasbefeuchters 1' angedeutet ist.

Die erste Teilmenge, die bei 14 den Gasbefeuchter 1' verläßt, wird über eine Pumpe 6, einem Wärmetauscher 7' zugeführt, der von der heißen Seite eines Wärmestromes, z. B. eines Synthesegases aus einem Methanolreaktor beaufschlagt wird, was wiederum durch eine Pfeillinie 9' angedeutet ist.

Die den Sumpf verlassende zweite Teilmenge wird über eine Pumpe einem zweiten Wärmetauscher 15 zugeleitet, der im Wärmestrom 9'—10' auf der kälteren Seite, d. h. stromabwärts, angeordnet ist, wie sich dies aus Fig. 2 ergibt. Dabei wird dieser Teilstrom bzw. Reststrom so stark erwärmt, daß er etwa die gleiche Temperatur aufweist, wie der bei 14 den Gasbefeuchter verlassende erste Teilstrom. Die beiden Teilmengen werden dann bei 16 vereinigt und dem letzten Wärmetauscher in diesem Kreislauf 7' zugeführt.

Aus Fig. 2 ergibt sich auch, daß das Zusatzwasser über 8' der zweiten Teilmenge bzw. der Restmenge zugespeist wird und damit der ersten Vorwärmung über den Wärmetauscher 15 mit unterworfen wird.

Fig. 3 zeigt ein weiteres Ausführungsbeispiel wiederum mit gleichen Bezugszeichen für prinzipiell gleiche Anlageteile. Der Unterschied besteht darin, daß neben der Enderhitzungsstufe durch den Wärmetauscher 7'' im gleichen Wärmestrom 9''—10'' zusätzlich zum Wärmetauscher 15 ein weiterer Wärmetauscher 17 vorgesehen ist, wobei hier prinzipiell die Strömungsführung der Teilmengen die gleiche ist wie beim Beispiel der Fig. 2, d. h. der kältesten Teilmenge bzw. Restmenge aus dem Sumpf 5'', wird bei 8'' das Zusatzwasser zugespeist, diese Mengen über den ersten Vorwärmer 15 geleitet und dann zusammen mit einer bei 18 den Gasbefeuchter verlassenden Teilmenge dem weiteren Wärmetauscher 17 zugeleitet, um dort weiter vorgewärmt zu werden. Zusammen mit der bei 19 den Gasbefeuchter 1'' verlassenden ersten Teilmenge wird die Gesamtwassermenge der Endheizstufe, d. h. dem Wärmetauscher 7'', zugeführt.

Natürlich sind die beschriebenen Ausführungsbeispiele noch in vielfacher Hinsicht abzuändern, ohne den Grundgedanken der Erfindung zu verlassen. So ist die Erfindung insbesondere nicht auf besondere bauliche Gestaltungen der im Verfahren eingesetzten Anlageteile beschränkt, auch nicht auf die in den Figuren wiedergegebene Prozeßführung. In der Bauart können auch Aggregate parallel statt hintereinander angeordnet sein u. dgl. mehr.

## Patentansprüche

1. Verfahren zum Befeuchten eines Gasstromes, bei dem aufgeheiztes Wasser zur Abgabe von Wasserdampf im Gegenstrom zum Gasstrom durch einen Gasbefeuchter und anschließend nach Einspeisung von der Menge des abgegebenen Wasserdampfes entsprechenden Zusatzwasser einer Aufheizstufe und zurück zum Gasbefeuchter im Kreislauf geführt wird, dadurch gekennzeichnet, daß Teilmengen des Kreislauf-Wassers in mehreren, hintereinanderliegenden Stufen dem Gasbefeuchter entnommen und die einzelnen Teilmengen einzeln und/oder gemeinsam erhitzt werden.

2. Verfahren nach Anspruch 1, dadurch gekennzeichnet, daß das Kreislauf-Wasser in zwei Stufen dem Gasbefeuchter entnommen wird, wobei die erste entnommene Teilmenge größer ist als die zweite Teilmenge.

3. Verfahren nach Anspruch 2, dadurch gekennzeichnet, daß die zweite Teilmenge einer Vorwärmstufe und anschließend zusammen mit der ersten Teilmenge einer Endheizstufe zugeführt wird.

4. Verfahren nach einem der vorangehenden Ansprüche, dadurch gekennzeichnet, daß der letzten aus dem Gasbefeuchter entnommenen Teilmenge die Zusatzwassermenge vor der ersten Vorwärmstufe zugeführt wird.

5. Verfahren nach einem der vorangehenden Ansprüche, dadurch gekennzeichnet, daß die Vorwärmstufe oder -stufen und die Endheizstufe als Wärmetauscher hintereinander in einem gemeinsamen Wärmestrom geschaltet sind.

6 Verfahren nach Anspruch 5, dadurch gekennzeichnet, daß die Teilmenge aus dem vorangehenden stromabwärts bezogen auf den Wärmestrom liegenden Wärmetauscher zusammen mit einer Teilmenge aus dem Gasbefeuchter dem nächsten stromaufwärtsliegenden Wärmetauscher zugeführt wird.

## Claims

1. A method of humidifying a gas flow wherein heated water, for the purpose of giving off water vapour, is circulated in counter-flow relationship to the gas flow through as gas humidifier and then, after feeding in make-up water corresponding to the amount of water vapour given off, it is circulated to a heating stage and back to the gas humidifier, characterized in that portions of the circulating water are taken from the gas humidifier in a plurality of successive stages and the individual portions are heated individually and/or jointly.

2. A method according to claim 1, characterized in that the circulating water is taken from the gas humidifier in two stages, wherein the first portion taken from the gas humidifier is larger than the second portion.

3. A method according to claim 2, characterized in that the second portion is passed to a preheating stage and then, in conjunction with the first portion, to a final heating stage.

4. A method according to one of the preceding claims, characterized in that the amount of make-up water is fed to the last portion taken from the gas humidifier, upstream of the first preheating stage.

5. A method according to one of the preceding claims, characterized in that the preheating stage or stages and the final heating stage are connected, as heat exchangers, in succession in a common heat flow.

6. A method according to claim 5, characterized in that the portion from the preceding heat exchanger which is downstream with respect to the heat flow is fed, together with a portion from the gas humidifier, to the next upstream-disposed heat exchanger.

## Revendications

1. Procédé pour humidifier un courant de gaz dans lequel on fait circuler de l'eau réchauffée pour céder de la vapeur d'eau à contre-courant du courant de gaz au travers d'un humidificateur à gaz puis, après introduction de la quantité d'eau de complément correspondant à la vapeur d'eau cédée, dans un stade de réchauffage et, en retour, à l'humidificateur à gaz, caractérisé en ce que l'on prélève des portions de l'eau de circulation en plusieurs étages successifs de l'humidificateur à gaz et on réchauffe les portions individuelles séparément et/ou en commun.

2. Procédé selon la revendication 1, caractérisé en ce que l'on prélève l'eau de circulation en deux stades dans l'humidificateur à gaz, la première portion prélevée étant plus forte que la deuxième.

3. Procédé selon la revendication 2, caractérisé en ce que l'on envoie la deuxième portion à un stade de chauffage préalable puis, avec la première portion, à un stade de chauffage final.

4. Procédé selon l'une des revendications qui précèdent, caractérisé en ce que l'on envoie la quantité d'eau de complément dans la dernière portion prélevée dans l'humidificateur à gaz, avant le premier stade de chauffage préalable.

5. Procédé selon l'une des revendications qui précèdent, caractérisé en ce que le ou les stades de chauffage préalables et le stade de chauffage final sont insérés sous forme d'échangeurs de chaleur successivement dans un courant de chaleur commun.

6. Procédé selon la revendication 5, caractérisé en ce que la portion sortant de l'avant-dernier échangeur de chaleur en aval dans le courant de chaleur est envoyée avec une portion sortant de l'humidificateur à gaz à l'échangeur de chaleur placé immédiatement après en amont du courant.

feuchtes Gas

FIG.1

3
4
1
10          7
9
2
Gas
Wärmeträger
5
6                    8       Zusatzwasser

feuchtes Gas        FIG.2

3'
4'
1'
10'      15      7'
9'
11
13
14          6          16
12
6
Gas
Wärmeträger
2'
5'
6          8'      Zusatzwasser

FIG. 3